# EUROPEAN PATENT SPECIFICATION

(11) **EP 1 835 856 B1**
(45) Date of publication and mention of the grant of the patent: **31.07.2013**
(21) Application number: 06700534.8
(22) Date of filing: 11.01.2006
(51) Int. Cl.: A61B 17/00

(54) **MEDICAL CLOSURE DEVICE**
MEDIZINISCHE DICHTUNGSVORRICHTUNG
DISPOSITIF D'OBTURATION MEDICALE

(30) Priority: 14.01.2005 US 643601 P
(43) Date of publication of application: 26.09.2007
(73) Proprietor: RADI MEDICAL SYSTEMS AB, 754 50 Uppsala (SE)
(72) Inventor: MATHISEN, Torbjörn, S-125 42 Älvsjö (SE); EGNELÖV, Per Radi Med.Syst.Co., Ltd. 154/7 Moo 9, Paklok Sub-District Phuket 83110 (TH)
(74) Representative: Holmberg, Nils Anders Patrik
(86) International application number: PCT/IB2006/000032
(87) International publication number: WO 2006/075228

(56) References cited:
- WO-A-95/20916
- WO-A-2004/012603
- WO-A-2005/002451

## Description

### FIELD OF THE INVENTION

The present invention relates generally to the field of sealing devices for the sealing of a percutaneous puncture in a vessel wall (for example, the wall of an artery), and in particular to the class of sealing devices that comprise an intra-arterial member and an extra-arterial member, which sandwich the vessel wall and are held together by a retaining member.

### BACKGROUND OF THE INVENTION

In the U.S. Patent No. 6,508,828, which is assigned to the present assignee, a sealing device is disclosed for sealing a puncture hole in a vessel wall. The sealing device comprises an inner sealing member, an outer member, and a retaining member. The inner sealing member is adapted to be positioned at the inner wall of a vessel, while the outer member is adapted to be positioned at the outer wall of the vessel. In use, the inner and outer members sandwich the vessel wall, and are held together by the retaining member to thereby seal the puncture hole in the vessel wall.

In U.S. Patent No. 6,596,012, which also is assigned to the present assignee, it is described how the sealing action of an inner sealing member can be improved by providing the inner sealing member with a rim portion that has a lower structural rigidity than a central portion of the inner sealing member.

Other examples of sealing devices that comprise an inner member and an outer member, which are held together by an elongated retaining member, such as a suture or filament, can be found in, for example, U.S. Patent Nos. 5,593,422 and 5,620,461. In U.S. Patent No. 5,342,393, the retaining member is in the form of a stem that extends from the inner member.

WO-2005/002451 relates to a wound closure and sealing device comprising en elongated member connected to a sealing element and configure to extend in an incision canal leading to a puncture in a vessel. Some parts of the device include a haemostatic material.

WO-95/20916 and WO-2004/012603 also relate to closure and sealing devices that include a haemostatic or coagulant material.

### SUMMARY OF THE INVENTION

Although at least a sealing device designed according to the teachings of U.S. Patent Nos. 6,508,828 and 6,596,012 in practice has proven to work very well, its sealing function can be improved. A general object of the present invention is therefore to provide a sealing device with an enhanced sealing capacity. Preferably, the invention should be applicable to an existing sealing device without significantly changing the design of the other components of the sealing device, or changing the practical handling of the sealing device.

The present invention is related to a sealing device comprising an intra-arterial (inner) member and an extra-arterial (outer) member, which are held together by a retaining member. In use, the inner member is introduced into the lumen of the vessel through a puncture hole in a vessel wall, and is then retracted until it is in close contact with the inner vessel wall. The retaining member, which is attached to the inner member, then extends through the puncture hole and holds the inner member tightly in a fixed position. The outer member is then advanced along the retaining member until the outer member is contacting the outside of the vessel wall. When the operation is completed, the outer and inner members will thereby sandwich the vessel wall and the puncture hole therein.

The actual sealing of the puncture hole can in principle be accomplished by two different mechanisms, either by clamping the vessel wall between the inner and outer members, or by the inner member alone. In the latter case, the outer member merely acts as a locking disc or member, which holds the inner sealing member in place. For the purpose of the present invention, it is rather irrelevant which one of these two effects actually accomplishes the sealing of the puncture hole. (For the sake of completeness, a sealing device comprising an intra-arterial anchor member and an extra-arterial sealing member, such that the sealing is accomplished outside the vessel wall, is not considered to fall within the scope of the present invention.)

According to an embodiment of the invention, the sealing performance of a sealing device comprising an inner member, an outer member and a retaining member can be improved by providing a haemostatic agent. The haemostatic agent is incorporated into a perforated or porous superficial structure of the outer member. The haemostatic agent is provided at the inner side of the outer member, i.e. at the side that faces a vessel wall, and will thereby chemically and/or biologically promote blood coagulation and wound healing, as a complement to the normal mechanical sealing action of the sealing device.

The invention is defined in the claims.

### BRIEF DESCRIPTION OF THE DRAWINGS

Fig. 1 is a schematic illustration of the components of a sealing device according to an embodiment of the present invention.
Fig. 2 shows the sealing device of Fig. 1 in a state corresponding to the completion of a medical sealing operation.
Fig. 3 is an enlarged view of a portion of the sealing device of Fig. 1 and illustrates how a haemostatic agent can be provided as an exterior layer on an outer member.
Fig. 4 illustrates how a haemostatic agent can be incorporated in the superficial structure of a porous or perforated outer member.
Fig. 5 is a schematic illustration of the components of a sealing device according to another embodiment of the present invention.
Fig. 6 is a schematic illustration of the components of a sealing device according to another embodiment of the present invention.
Fig. 7 is a schematic illustration of the components of a sealing device according to another embodiment of the present invention.
Fig. 8 is a schematic illustration of components of a sealing device according to another embodiment of the present invention.

### DETAILED DESCRIPTION OF EMBODIMENTS OF THE INVENTION

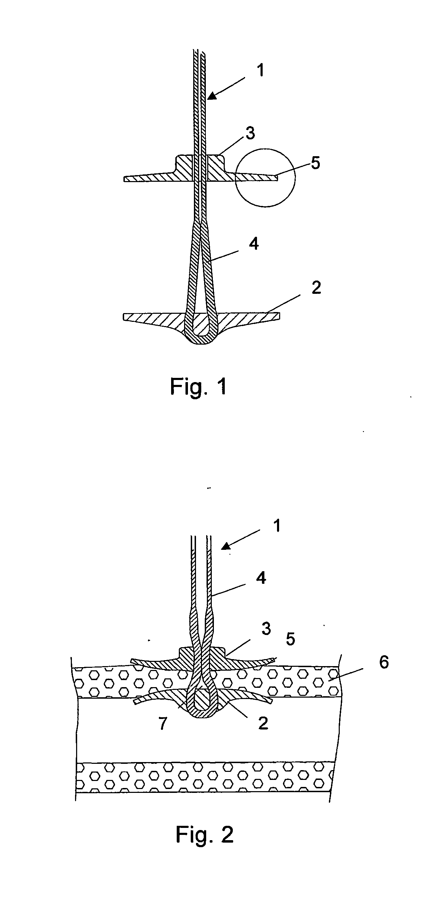

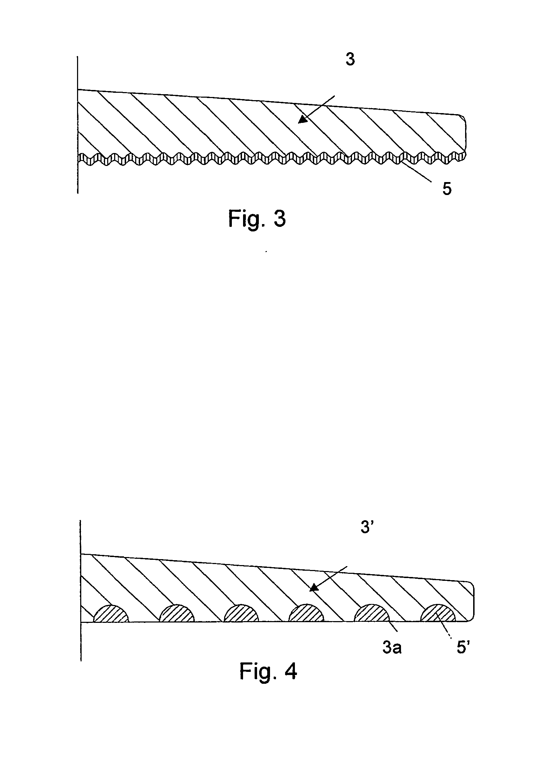

A sealing or closure device 1 according to the present invention is schematically illustrated in Fig. 1. The sealing device 1 comprises an inner member 2 and an outer member 3, which are held together by an elongated retaining member 4. The inner member 2, the retaining member 4 and basically also the outer member 3 are all of previously known designs, examples of which can be found in the above-referenced patents. The retaining member 4 is attached to the inner member 2, and extends through a hole in the outer member 3. During the positioning operation of the sealing device 1, the outer member 3 can thereby slide along the retaining member 4 into abutment against the outer surface of a vessel. Unlike the previously known sealing devices, the sealing device 1 comprises further a haemostatic agent 5, which in this embodiment has the form of a thin exterior layer 5 provided at the inner side of the outer member 3. The encircled portion of the outer member 3 is depicted in Fig. 3, and will be further discussed in conjunction with Fig. 3 below.

The functions of the sealing device 1 and the haemostatic layer 5 are illustrated in Fig. 2, where the sealing device 1 has been positioned around a vessel wall 6 in order to close a puncture hole 7 therein. The figure illustrates that the inner member 2 is positioned inside the vessel at an interior surface of the vessel wall 6, while the outer member 3 is positioned at an exterior surface of the vessel wall 6, and that the retaining member 4 extends through the puncture hole 7 in the vessel wall 6, such that the vessel wall 6 is sandwiched between the inner member 2 and the outer member 3. In this embodiment, inner member 2 contacts the interior surface of the vessel wall 6 and seals hole 7. In this example, a portion of the retaining member 4 has been provided with an enlarged thickness, such that the outer member 3 is held in a fixed position by the friction acting between the retaining member 4 and the outer member 3. It should, however, be understood that other ways of securing an outer member to a retaining member can be provided, for example by having a retaining member in the form of a saw-toothed stem. Although not shown in the figures, it is preferred that the curvatures of the inner and outer members are adapted to the curvature of the vessel wall.

Fig. 2 shows that the haemostatic layer 5 is provided at the side of the outer member 3 that faces the vessel wall 6. This side is herein referred to as the inner side or inner surface of an outer member. The haemostatic layer 5 comprises a suitable haemostatic agent that promotes the coagulation process and thereby facilitates healing of the puncture hole 7 in the vessel wall 6. The haemostatic layer will thereby complement the mechanical sealing action of the inner and outer members 2, 3.

An enlargement of the encircled portion of the outer member 3 shown in Fig. 1 is depicted in Fig. 3, where it is clearly seen that the haemostatic layer 5 is arranged as a thin exterior coating on the inner surface of the outer member 3. In order to improve the adhesion of a haemostatic agent to an inner side of an outer member, the corresponding inner surface can be provided with a roughened texture, as is indicated in Fig. 3.

Another way of providing an outer member with a haemostatic agent is shown in Fig. 4, where a portion of an outer member 3' is illustrated. In this embodiment, the outer member 3' has been provided with a number of small cavities 3a, which are filled with a haemostatic agent 5'. An advantage with this way of arranging a haemostatic agent is that the haemostatic agent is less likely to be peeled off and stick to, for example, an introducer device that is used to introduce and position a sealing device. Instead of rather shallow cavities, an inner side of an outer member can be provided with deeper perforations or holes. Such perforations or holes can be through-going, i.e. extend all the way to the outer side of an outer member. As an alternative, an outer member can exhibit a porous superficial texture, such that a haemostatic agent can be incorporated into the porous matrix of the outer member. The porosity of an outer member can also extend deeper into the outer member, and the outer member can be a porous outer member. Preferably the outer member as well as the inner member is made from a resorbable material, as is well-known in the art.

Non-limiting examples of haemostatic agents that can be used together with an outer member according to the invention are: collagen, chitin, chitosan, thrombin, prothrombin, gelatine, oxidized regenerated cellulose, aprotinin, tranexamic acid, aminocaproic acid, desmopressin, vitamin K, factor XIIa, factor Va, factor VIIa, factor VIII, factor Xa, vasopressin, and conjugated oestrogen, or combinations thereof. To retain the haemostatic agent various forms of water-soluble polymers or carbohydrates can be used as an excipient for the haemostatic agent. Optionally, a haemostatic agent that is incorporated in an outer member in, for example, the ways described in conjunction with Fig. 3 and Fig. 4 can also be covered and protected by a thin layer of a preferably quickly dissolvable material, such as hemicellulose.

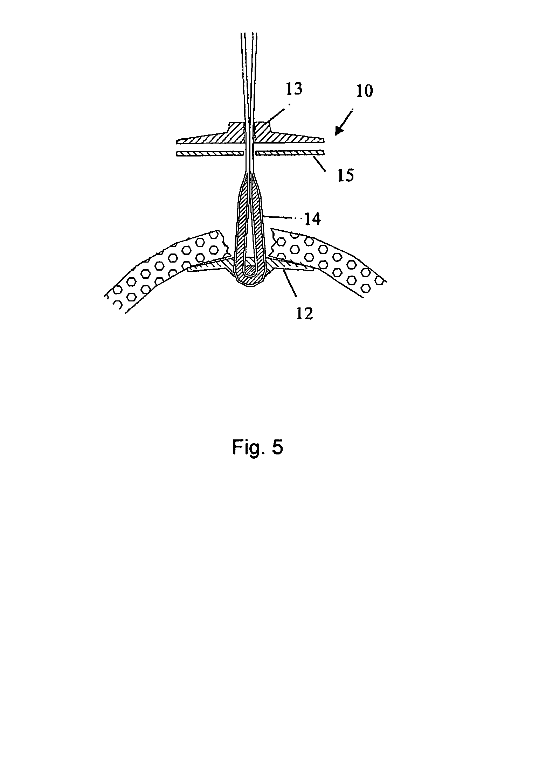

Figures 5-8 are schematic diagrams illustrating components of sealing devices according to other embodiments of the present invention.

Figure 5 shows a sealing or closure device 10 having an inner member 12, an outer member 13, and a retaining member 14. A haemostatic layer 15 is provided in the form of a washer or disk. This location of haemostatic layer 15 can be used, for example, to provide the haemostatic effect in a direction parallel to retaining member 14. The haemostatic layer 15 can also be provided on top of outer member 13.

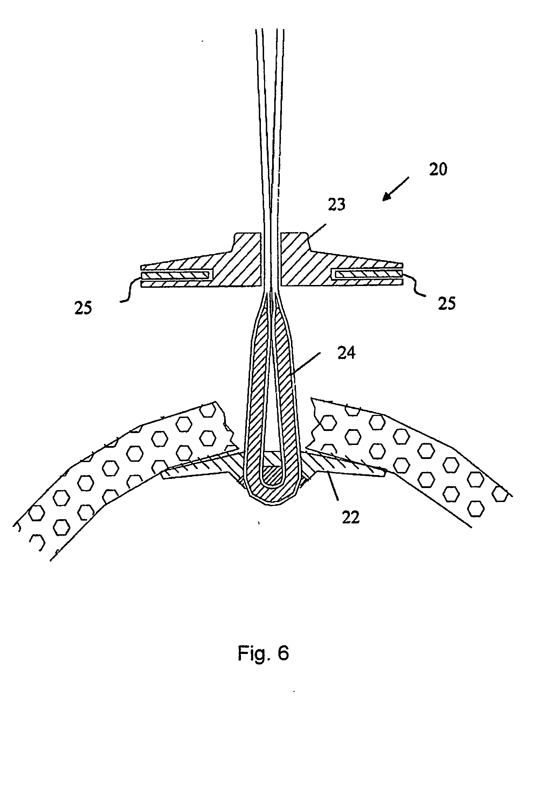

Figure 6 depicts a sealing or closure device 20 having an inner member 22, an outer member 23, and a retaining member 24. A haemostatic layer 25 is inserted in outer member 23. Such an arrangement can be used, for example, to cause the haemostatic effect in a direction perpendicular to retaining member 24.

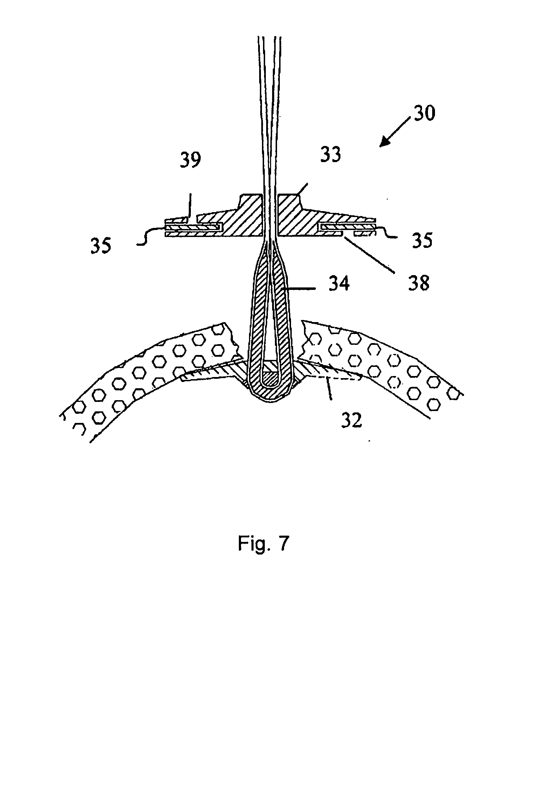

Figure 7 depicts a sealing or closure device 30 having an inner member 32, an outer member 33, and a retaining member 34. A haemostatic layer 35 is inserted into the outer member 33, similar to the Figure 6 embodiment. However, in the Figure 7 embodiment, the outer member 33 is provided with holes 38 and/or 39 in order to direct the haemostatic agent toward a particular location.

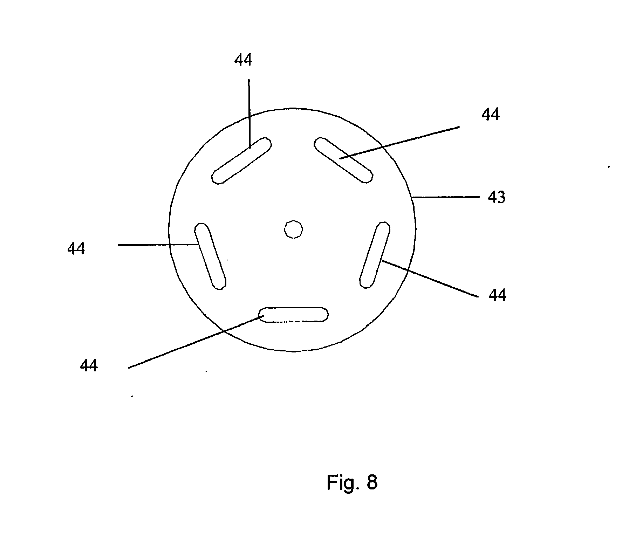

Figure 8 depicts an outer member 43 having a retaining member 44, such as a suture. In the Figure 8 embodiment, retaining member 44 is impregnated with a haemostatic agent.

Although the present invention has been described with reference to specific embodiments, also shown in the appended drawings, it will be apparent for those skilled in the art that many variations and modifications can be done within the scope of the invention as described in the specification and defined with reference to the claims below.

## Claims

1. A medical sealing device for the sealing of a puncture hole in a vessel wall, comprising: an inner member (2), which is adapted to be positioned at an interior surface of the vessel wall; an outer member (3,3'), which is adapted to be positioned outside the vessel wall and provided with an inner surface or inner side adapted to face the wall; the inner member and the outer member being held together by a retaining member (1); wherein the sealing device further comprises a haemostatic agent (5) at the outer member, **characterized in that** the inner side of the outer member is provided with perforations or cavities (3a) or a roughened texture, in which the haemostatic agent (5,5') is arranged, or the outer member has a porous inner surface, in which the haemostatic agent is incorporated.

2. A medical sealing device for the sealing of a puncture hole in a vessel wall, comprising: an inner member (2), which is adapted to be positioned at an interior surface of the vessel wall; an outer member (3,3'), which is adapted to be positioned outside the vessel wall and provided with an inner surface or inner side adapted to face the wall; the inner member and the outer member being held together by a retaining member (1); wherein the sealing device further comprises a haemostatic agent (5) at the outer member, **characterized in that** the inner side of the outer member is provided with cavities (3a), in which the haemostatic agent (5,5') is arranged.

## Patentansprüche

1. Medizinische Versiegelungseinrichtung zum Versiegeln eines Punktionslochs in einer Gefäßwand, mit: einem inneren Element (2), das eingerichtet ist, an einer Innenfläche der Gefäßwand positioniert zu werden, einem äußeren Element (3, 3'), das eingerichtet ist, außerhalb der Gefäßwand positioniert zu werden und mit einer inneren Fläche oder Innenseite bereitgestellt ist, die eingerichtet ist, der Wand zugewandt zu sein, wobei das innere Element und das äußere Element durch ein Rückhalteelement (1) zusammengehalten werden und die Versiegelungseinrichtung des weiteren an dem äußeren Element ein Hämostyptikum (5) aufweist, **dadurch gekennzeichnet, dass** die Innenseite des äußeren Elements mit Perforationen oder Aushöhlungen (3a) oder einer rauen Oberflächenstruktur bereitgestellt ist, in der das Hämostyptikum (5, 5') angeordnet ist, oder das äußere Element eine poröse innere Fläche aufweist, in der das Hämostyptikum eingearbeitet ist.

2. Medizinische Versiegelungseinrichtung zum Versiegeln eines Punktionslochs in einer Gefäßwand, mit: einem inneren Element (2), das eingerichtet ist, an einer inneren Fläche der Gefäßwand positioniert zu werden, einem äußeren Element (3, 3'), das eingerichtet ist, außerhalb der Gefäßwand positioniert zu werden und mit einer inneren Fläche oder Innenseite bereitgestellt ist, die eingerichtet ist, der Wand zugewandt zu sein, wobei das innere Element und das äußere Element durch ein Rückhalteelement (1) zusammengehalten werden und die Versiegelungseinrichtung des Weiteren an dem äußeren Element ein Hämostyptikum (5) aufweist, **dadurch gekennzeichnet, dass** die Innenseite des äußeren Elements mit Aushöhlungen (3a) bereitgestellt ist, in denen das Hämostyptikum (5, 5') angeordnet ist.

## Revendications

1. Dispositif de scellement médical pour le scellement d'un trou de piqûre dans une paroi de vaisseau, comprenant : un élément intérieur (2), qui est adapté de façon à être positionné à une surface intérieure de la paroi de vaisseau ; un élément extérieur (3, 3'), qui est adapté de façon à être positionné à l'extérieur de la paroi de vaisseau et qui est muni d'une surface intérieure ou d'un côté intérieur adapté de façon à faire face à la paroi ; l'élément intérieur et l'élément extérieur étant maintenus réunis l'un à l'autre par un élément de maintien (1) ; dans lequel le dispositif de scellement comprend de plus un agent hémostatique (5) au niveau de l'élément extérieur, **caractérisé en ce que** le côté intérieur de l'élément extérieur est muni de perforations ou de cavités (3a) ou a une texture rugueuse, où est disposé l'agent hémostatique (5, 5'), ou **en ce que** l'élément extérieur comporte une surface intérieure poreuse, dans laquelle l'agent hémostatique est incorporé.

2. Dispositif de scellement médical pour le scellement d'un trou de piqûre dans une paroi de vaisseau, comprenant : un élément intérieur (2), qui est adapté de façon à être positionné à une surface intérieure de la paroi de vaisseau ; un élément extérieur (3, 3'), qui est adapté de façon à être positionné à l'extérieur de la paroi de vaisseau et qui est muni d'une surface intérieure ou d'un côté intérieur adapté de façon à faire face à la paroi ; l'élément intérieur et l'élément extérieur étant maintenus réunis l'un à l'autre par un élément de maintien (1) ; dans lequel le dispositif de scellement comprend de plus un agent hémostatique (5) au niveau de l'élément extérieur, **caractérisé en ce que** le côté intérieur de l'élément extérieur est muni de cavités (3a), dans lesquelles est disposé l'agent hémostatique (5, 5').
